# EUROPEAN PATENT APPLICATION

(11) **EP 3 750 427 A1**
(43) Date of publication of application: **16.12.2020**
(21) Application number: 19179935.2
(22) Date of filing: 13.06.2019
(51) Int. Cl.: A24F 47/00

(54) **A SYSTEM AND METHOD FOR MANAGING A SMOKING SUBSTITUTE DEVICE**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

A smoking substitute device is provided comprising a control unit, a communications interface, and a memory configured to store firmware for the control of the smoking substitute device. The firmware is encrypted by a first encryption key. The control unit is configured to encrypt data relating to the use of the smoking substitute device using a second encryption key and record a data log in the memory, comprising the encrypted data relating to the usage of the smoking substitute device. The smoking substitute device is configured to transmit information to and receive information from a mobile device via the communications interface. The information transmitted to or received from the mobile device via the communications interface is encrypted using a third encryption key.

## Description

### TECHNICAL FIELD

The present invention relates to smoking substitute devices. In particular, although not exclusively, it relates to the management of security for smoking substitute devices.

### BACKGROUND

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful byproducts. There have been proposed various smoking substitute devices in order to avoid the smoking of tobacco.

Such smoking substitute devices can form part of nicotine replacement therapies aimed at people who wish to stop smoking and overcome a dependence on nicotine.

Smoking substitute devices may comprise electronic systems that permit a user to simulate the act of smoking by producing an aerosol (also referred to as a "vapour") that is drawn into the lungs through the mouth (inhaled) and then exhaled. The inhaled aerosol typically bears nicotine and/or flavourings without, or with fewer of, the odour and health risks associated with traditional smoking.

In general, smoking substitute devices are intended to provide a substitute for the rituals of smoking, whilst providing the user with a similar experience and satisfaction to those experienced with traditional smoking and tobacco products. Some smoking substitute systems use smoking substitute articles (also referred to as a "consumables") that are designed to resemble a traditional cigarette and are cylindrical in form with a mouthpiece at one end.

The popularity and use of smoking substitute devices has grown rapidly in the past few years. Although originally marketed as an aid to assist habitual smokers wishing to quit tobacco smoking, consumers are increasingly viewing smoking substitute devices as desirable lifestyle accessories. Some smoking substitute devices are designed to resemble a traditional cigarette and are cylindrical in form with a mouthpiece at one end. Other smoking substitute devices do not generally resemble a cigarette (for example, the smoking substitute device may have a generally box-like form).

There are a number of different categories of smoking substitute devices, each utilising a different smoking substitute approach. A smoking substitute approach corresponds to the manner in which the substitute system operates for a user.

One approach for a smoking substitute device is the so-called "vaping" approach, in which a vapourisable liquid, typically referred to (and referred to herein) as "e-liquid", is heated by a heating device to produce an aerosol vapour which is inhaled by a user. An e-liquid typically includes a base liquid as well as nicotine and/or flavourings. The resulting vapour therefore typically contains nicotine and/or flavourings. The base liquid may include propylene glycol and/or vegetable glycerin.

A typical vaping smoking substitute device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

Vaping smoking substitute devices can be configured in a variety of ways. For example, there are "closed system" vaping smoking substitute devices which typically have a sealed tank and heating element which is pre-filled with e-liquid and is not intended to be refilled by an end user. One subset of closed system vaping smoking substitute devices include a main body which includes the power source, wherein the main body is configured to be physically and electrically coupled to a consumable including the tank and the heating element. In this way, when the tank of a consumable has been emptied, the main body can be reused by connecting it to a new consumable. Another subset of closed system vaping smoking substitute devices are completely disposable, and intended for one-use only.

There are also "open system" vaping smoking substitute devices which typically have a tank that is configured to be refilled by a user, so the device can be used multiple times.

An example vaping smoking substitute device is the myblu™ e-cigarette. The myblu™ e-cigarette is a closed system device which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heating device, which for this device is a heating filament coiled around a portion of a wick which is partially immersed in the e-liquid. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

Another example vaping smoking substitute device is the blu PRO™ e-cigarette. The blu PRO™ e-cigarette is an open system device which includes a main body, a (refillable) tank, and a mouthpiece. The main body and tank are physically and electrically coupled together by screwing one to the other. The mouthpiece and refillable tank are physically coupled together by screwing one of the other, and detaching the mouthpiece from the refillable tank allows the tank to be refilled with e-liquid. The device is activated by a button on the main body. When the device is activated, electrical energy is supplied from the power source to a heating device, which heats e-liquid from the tank to produce a vapour which is inhaled by a user through the mouthpiece.

Another approach for a smoking substitute system is the so-called Heated Tobacco ("HT") approach in which tobacco (rather than an "e-liquid") is heated or warmed to release vapour. HT is also known as "heat not burn" ("HNB"). The tobacco may be leaf tobacco or reconstituted tobacco. The vapour may contain nicotine and/or flavourings. In the HT approach the intention is that the tobacco is heated but not burned, i.e. the tobacco does not undergo combustion.

A typical HT smoking substitute system may include a device and a consumable. The consumable may include the tobacco material. The device and consumable may be configured to be physically coupled together. In use, heat may be imparted to the tobacco material by a heating element of the device, wherein airflow through the tobacco material causes components in the tobacco material to be released as vapour. A vapour may also be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the consumable (entrained in the airflow) from the location of vaporisation to an outlet of the consumable (e.g. a mouthpiece), the vapour cools and condenses to form an aerosol for inhalation by the user. The aerosol will normally contain the volatile compounds.

In HT smoking substitute systems, heating as opposed to burning the tobacco material is believed to cause fewer, or smaller quantities, of the more harmful compounds ordinarily produced during smoking. Consequently, the HT approach may reduce the odour and/or health risks that can arise through the burning, combustion and pyrolytic degradation of tobacco.

There may be a need for improved design of smoking substitute systems, in particular HT smoking substitute systems, to enhance the user experience and improve the function of the HT smoking substitute system.

An example of the HT approach is the IQOS™ smoking substitute device from Philip Morris Ltd. The IQOS™ smoking substitute device uses a consumable, including reconstituted tobacco located in a wrapper. The consumable includes a holder incorporating a mouthpiece. The consumable may be inserted into a main body that includes a heating device. The heating device has a thermally conductive heating knife which penetrates the reconstituted tobacco of the consumable, when the consumable is inserted into the heating device. Activation of the heating device heats the heating element (in this case a heating knife), which, in turn, heats the tobacco in the consumable. The heating of the tobacco causes it to release nicotine vapour and flavourings which may be drawn through the mouthpiece by the user through inhalation.

A second example of the HT approach is the device known as "Glo"™ from British American Tobacco p.l.c. Glo™ comprises a relatively thin consumable. The consumable includes leaf tobacco which is heated by a heating device located in a main body. When the consumable is placed in the main body, the tobacco is surrounded by a heating element of the heating device. Activation of the heating device heats the heating element, which, in turn, heats the tobacco in the consumable. The heating of the tobacco causes it to release nicotine vapour and flavourings which may be drawn through the consumable by the user through inhalation. The tobacco, when heated by the heating device, is configured to produce vapour when heated rather than when burned (as in a smoking apparatus, e.g. a cigarette). The tobacco may contain high levels of aerosol formers (carrier), such as vegetable glycerine ("VG") or propylene glycol ("PG").

The present inventor(s) have observed that most smoking substitute devices currently on the market are configured to operate in isolation of other devices, which limits the functions the smoking substitute devices can perform.

The present inventor(s) have observed that it is important for security to be considered in relation to smoking substitute devices. This can be particularly important as the number of functional components provided in smoking substitute devices, and as the data stored about component operation and about the user and his or her usage behaviour, increases. It can be particularly important for network-enabled smoking substitute devices that are configured to wirelessly communicate with one or more other devices and/or with a server such as an application server.

The present invention has been devised in light of the above considerations.

### Summary of the Invention

At its most general, the present invention provides a device, system and method that enables secure and reliable storage and transmission of data for a smoking substitute device. The present inventor(s) have identified three important types (or facets, or streams) of data relating to a smoking substitute that should be protected, for device security and user peace of mind. Those data types are:
(1) firmware stored on the smoking substitute device,
(2) usage data recorded during operation of the smoking substitute device, and
(3) data to be transmitted wirelessly from the smoking substitute device to a mobile device or remote server.

According to the invention, each of these data types is assigned its own encryption key. The three encryption keys assigned to a particular smoking substitute device will be different to one another and each individual smoking substitute device may have its own unique combination of three encryption keys.

The three encryption keys will be stored securely within the memory of the smoking substitute device. For example, they can be stored in a portion of the memory that is ringfenced from other portions of the memory and which cannot be read by another device, such as a mobile device with which the smoking substitute device can wirelessly communicate. They may be stored in a dedicated hardware security module or within a protected region of the memory. The three encryption keys may be stored separately to one another, in different respective secure portions of the memory, to further enhance the robustness of the security architecture of the smoking substitute device.

The three encryption keys may also be stored in a server, for example an application server, with which the smoking substitute device can be configured to communicate, either directly or via a mobile device on which an application is being run. The server may be configured to decrypt encrypted data, for example encrypted data that the smoking substitute device has wirelessly transmitted to the mobile device, and the server may provide the decrypted data to the mobile device. However, the server will not provide an encryption key itself to the mobile device or to any other device.

The invention relates in particular to network-enabled smoking substitute devices, where in a network-enabled device has a wireless interface for communicating with the wireless interface of another device. For example, the other device may be a mobile device such as a mobile phone, smartphone, laptop computer or tablet computer or a television or gaming device. The wireless interface may comprise any suitable type of wireless communication interface, or terminal, for example a Wi-Fi or Bluetooth™ or Bluetooth™ Low Energy (BLE) interface.

The invention thus provides an improved security architecture for a smoking substitute device that ensures that the data stored within the device and the transfer of that data is secure and inaccessible to a third-party device, except where the user permits some access, for example by inputting commands into an application running on a mobile device. The security architecture has an increased level of security, as compared to known smoking substitute devices, because it provides three unique encryption keys, per device. If a device were to be "hacked" (i.e. if the security architecture were to be penetrated by a non-authorised user) and the encryption keys discovered, the "hacker" would only have discovered the encryption keys that are applicable to that sole smoking substitute device and all other devices of its type (e.g. of the same make, model, brand etc) would remain secure. Moreover, each of three data streams - relating to each of the firmware, the data logs and the wireless messaging respectively - would have to be individually hacked, in order to obtain all the encryption key for a single device. The effort involved in doing this, for an individual device, will act as a deterrent for many potential hackers.

According to a first aspect of the invention, there is provided smoking substitute device comprising: a control unit; a communications interface; and a memory configured to store firmware for controlling the smoking substitute device. The firmware is encrypted by a first encryption key. The control unit is configured to: encrypt data relating to the use of the smoking substitute device (also referred to herein as usage data) using a second encryption key; and record in the memory a data log comprising the encrypted data relating to the use of the smoking substitute device. The smoking substitute device is configured to transmit information to and receive information from a mobile device via the communications interface, wherein the information transmitted to or received from the mobile device via the communications interface is encrypted using a third encryption key.

The first encryption key, second encryption key and third encryption keys may be different from one another. Moreover, the particular combination of first encryption key, second encryption key and third encryption keys that is assigned to a particular respective smoking substitute device may be unique to that device alone. Therefore, if the smoking substitute device is a first smoking substitute device, within a plurality of similar smoking substitute devices, the first, second and third encryption keys for the first smoking substitute device may be different to the first, second and third encryption keys for each of the respective other smoking substitute devices, within the plurality of smoking substitute devices.

The first, second and third encryption keys may each be stored in a secure memory location (also referred to herein as a secure region in the memory) within the smoking substitute device. The first, second and third encryption keys may be stored in the same secure memory location, or in respective independent secure memory locations. The secure memory location may be a non-readable portion of memory, e.g. to which access is controlled by a memory protection unit operating in any conventional manner. Alternatively the secure memory location may be a hardware security module provided within the smoking substitute device.

The firmware may comprise software that is stored on the smoking substitute device and that provides the control instructions for operation of the device's specific hardware components. The control unit of the smoking substitute device can be configured to run the firmware, to control operation of the device. The control unit may be configured to run the firmware by decrypting the encrypted firmware stored in the memory using the first encryption key stored in the secure memory location.

According to an embodiment, the control unit may access the first encryption key, and/or may decrypt the firmware, in response to a command being received at the communications interface of the smoking substitute device from a corresponding communications interface of a mobile device with which the smoking substitute device wirelessly communicates. For example, the mobile device may transmit a command from an application running on the mobile device, wherein the command may have been generated by software components of the application and/or may comprise a user command that has been input to the application via a user interface of the mobile device.

The usage data may comprise one or more or a portion of one or more data logs. Each data log may comprise a record of data, stored within the memory of the smoking substitute device. A data log may be a list, a grouping or any other suitable type of record. A data log may be stored permanently or temporarily, for example using a buffer. The data that is encrypted using the second encryption key, and stored within a data log, comprises data relating to the use of the smoking substitute device. Data relating to the use of the smoking substitute device may comprise data pertaining to one or more of a plurality of components within the device and/or statistical data relating to user behaviour with respect to the device.

For example, the data that is encrypted using the second encryption key may relate to any of: a battery or other power source, an air flow sensor, a wireless interface, a position or orientation sensor such as an accelerometer or gyroscope, a charging port via which the device can be electrically recharged, or a coupling portion that interfaces with a consumable , wherein the coupling portion usually comprises a mechanical interface and an electrical interface via which power can be supplied to the consumable. The data may comprise an output from one or more of the sensors, which can be used either in isolation or in combination with other data in order to determine control instructions for the device. For example, if the data comprises output voltage levels from the battery, which are indicative of battery power level, such data can be used by the control unit to determine when the battery level is reaching a low threshold level at which operation of all of the components within the device may not be possible and thus at which operation of certain components may have to be temporarily compromised or suspended. For example, if the data comprises a detection output from the air flow sensor, this may be understood by the control unit to comprise an indication that and inhale action is being carried out using the device. The data that is logged and encrypted using the second encryption key may comprise time components such as indicators of when inhalations took place and their duration.

The information that is transmitted to or received from a mobile device, via the communications interface of the smoking substitute device, and that is encrypted using a third encryption key, may be of a number of different types. For example, the information transmitted to the mobile device may comprises the data relating to the use of the smoking substitute device encrypted by the second encryption key, e.g. any of the usage data referred to above. In other examples, the information transmitted or received via the communications interface may comprise a command issued to the mobile device or to an application that is running on the mobile device, or a request from the mobile device or from an application that is running on the mobile device.

The control unit of the smoking substitute device may be configured to encrypt the information that is to be transmitted to the mobile device, via the communications interface, using the third encryption key that is stored in the secure memory location. The control unit may be further configured to decrypt information received, by the communications interface, from a mobile device or application, using the third encryption key that is stored in the secure memory location.

The smoking substitute device may be configured to transmit information to and receive information from a server, wherein the information transmitted to or received from the server via the communications interface is encrypted using the third encryption key. The transmission and receipt of encrypted information to and from the server may be direct or may be via an intermediate such as a mobile device with which the smoking substitute device is configured to wirelessly communicate.

According to a second aspect of the invention, there is provided a system for managing a smoking substitute device, the system comprising a mobile device in network communication with a remote server, and a smoking substitute device. The mobile device comprises: a first control unit; a first communications interface; and a first memory. The smoking substitute device comprises: a second control unit; a second communications interface; and a second memory configured to store firmware for controlling the smoking substitute device. The firmware is encrypted by a first encryption key. The second control unit is configured to: encrypt data relating to the use of the smoking substitute device using a second encryption key; and record in the second memory a data log comprising the encrypted data relating to the use of the smoking substitute device. The mobile device and the smoking substitute device are in communication via the first communications interface and second communications interface to exchange information therebetween. The information exchanged between the mobile device and the smoking substitute device is encrypted using a third encryption key. The system may comprise the server, which may be, for example, an application server. An application running on the mobile device may be configured to communicate with the server, via a network. The server may utilise a virtual memory means such as cloud storage, for example.

The mobile device may be configured to: transmit encrypted information received from the smoking substitute device to the server; and receive, from the server, encrypted data to be transmitted to the smoking substitute device.. The server may be configured to decrypt the encrypted information received from the mobile device, using the third encryption key stored on the server. The server may process the decrypted information to determine data, commands or other information to be returned to the mobile device (e.g. for display on the application) or to be relayed to the smoking substitute device. Information for the smoking substitute device that is sensitive (e.g. firmware updates or the like) may be encrypted with the third encryption key so that it remains encrypted during relay via the mobile device.

The three encryption keys for the smoking substitute device should not be stored on, transmitted to or otherwise accessible by a mobile device, with which the smoking substitute device and/or the sever wirelessly communicates, or by any other third-party device. If the smoking substitute device is sending a command or other information to the mobile device, which is encrypted using the second encryption key, the mobile device will have to transmit that encrypted command or other information to the server, for it to be decrypted and sent back to the mobile device. It is possible that the mobile device will also have its own encryption keys that is applies to data transmissions between the mobile device and the server. However, any such mobile device encryption keys will be independent from the three encryption keys that have been implemented for the smoking substitute device.

The information transmitted to the mobile device from the smoking substitute device comprises the data relating to the use of the smoking substitute device encrypted by the second encryption key. The server may thus possess the third encryption key to decrypt the transmitted encrypted information received by the mobile device from the smoking substitute device, and the second encryption key to decrypt the data relating to the use of the smoking substitute device. The server may store all of the first encryption key, second encryption key, and third encryption key. For example, the server may be configured to transmit a firmware update to the smoking substitute device, and wherein the firmware update is encrypted with the first encryption key.

The smoking substitute device may be configured to transmit information to and receive information from the server, wherein the information transmitted to or received from the server, from or to the smoking substitute device, is encrypted using the third encryption key. The server may be configured to store the first encryption key, second encryption key, and third encryption key. The server may be configured to decrypt the information received from the smoking substitute device, using the third encryption key stored on the server, and is to transmit the decrypted information to the mobile device. In this manner, therefore, the encrypted data may bypass the mobile device and be submitted directly to the server, for use by the server and/or for decryption, after which, if appropriate, the decrypted data may be sent to the mobile device.

In another aspect, the invention provides a method of managing a smoking substitute device, wherein the smoking substitute device comprises a control unit, a communications interface, and a memory configured to store firmware for controlling the smoking substitute device, the method comprising: encrypting the firmware using a first encryption key; encrypting data relating to use of the smoking substitute device using a second encryption key; recording, in the memory, a data log comprising the encrypted data relating to the use of the smoking substitute device; encrypting information that is to be transmitted to a mobile device, via the communications interface, using a third encryption key; and transmitting encrypted information to a mobile device, via the communications interface. The method may be a computer implemented method. It may be implemented by the control unit of the smoking substitute device which may be, for example, a microprocessor.

The smoking substitute device may be configured to form a paired or bonded wireless communication link with a mobile device, wherein signals may be transmitted between the smoking substitute device and a mobile device via the paired or bonded wireless communication link. The process for pairing or bonding the two devices may comprise any suitable security protocol, for example the identification of each to the other and the sharing of encryption keys. If the smoking substitute device does form a paired or bonded communication link with a mobile device, the encryption key that it provides to the mobile device for pairing should be distinct from the three encryption keys detailed herein, that are specifically directed to encrypting the firmware, data log and transmitted and received messages for the smoking substitute device. Moreover, those three encryption keys should not be submitted to or stored on a mobile device, even if it is securely paired or bonded to the smoking substitute device, for wireless communication.

The method may include transmitting, from the mobile device to a remote server, encrypted information received from the smoking substitute device; and receiving, by the mobile device from the remote server, encrypted data to be transmitted to the smoking substitute device.

Although three encryption keys are detailed herein, it is possible that more than three encryption keys may be used, each directed to encrypting a different respective data facet or datastream for a smoking substitute device.

The invention includes the combination of the aspects and preferred features described except where such a combination is clearly impermissible or expressly avoided.

The skilled person will appreciate that except where mutually exclusive, a feature or parameter described in relation to any one of the above aspects may be applied to any other aspect. Furthermore, except where mutually exclusive, any feature or parameter described herein may be applied to any aspect and/or combined with any other feature or parameter described herein.

### Summary of the Figures

Embodiments and experiments illustrating the principles of the invention will now be discussed with reference to the accompanying figures in which:
**Figure 1** shows an example system for managing a smoking substitute device.
**Figure 2(a)** shows an example smoking substitute device for use as the smoking substitute device in the system of Fig. 1.
**Figure 2(b)** shows the main body of the smoking substitute device of Fig. 2(a) without the consumable.
**Figure 2(c)** shows the consumable of the smoking substitute device of Fig. 2(a) without the main body.
**Figure 3(a)** is a schematic view of the main body of the smoking substitute device of Fig. 2(a) that is an embodiment of the invention.
**Figure 3(b)** is a schematic view of the consumable of the smoking substitute device of Fig. 2(a).
**Figure 4** is a flow diagram of encryption operations carried out by the control unit of a smoking substitute device, according to an embodiment of the present invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

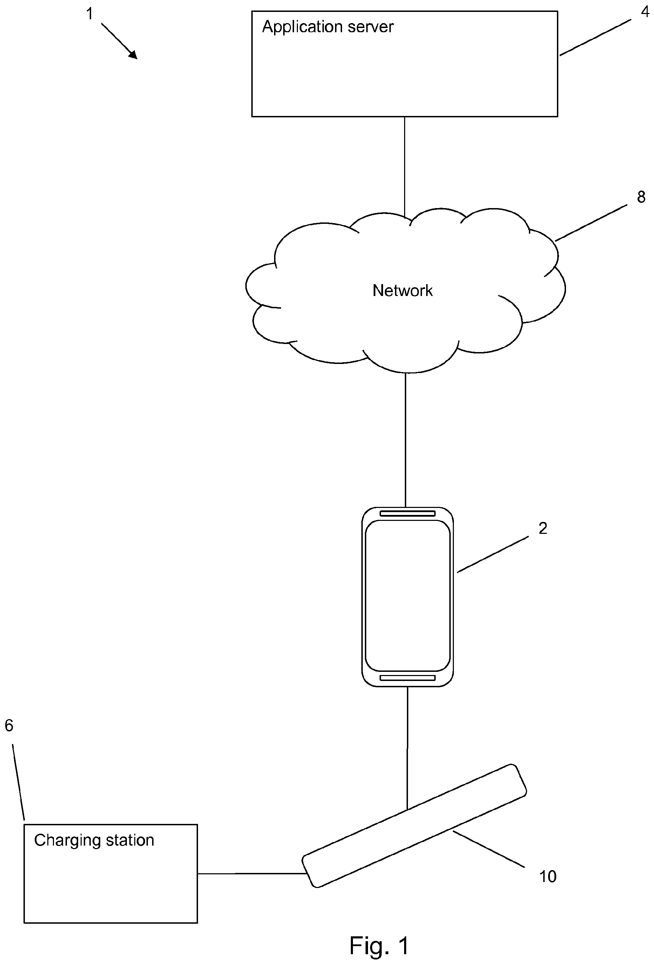

Fig. 1 shows an example system 1 for managing a smoking substitute device 10.

The system 1 as shown in Fig. 1 includes a mobile device 2, an application server 4, an optional charging station 6, as well as the smoking substitute device 10.

The smoking substitute device 10 is configured to communicate wirelessly, e.g. via Bluetooth™, with an application (or "app") installed on the mobile device 2, e.g. via a suitable wireless interface (not shown) on the mobile device 2. The mobile device 2 may be a mobile phone, for example. The application on the mobile phone is configured to communicate with the application server 4, via a network 8. The application server 4 may utilise cloud storage, for example.

The network 8 may include a cellular network and/or the internet.

A skilled person would readily appreciate that the mobile device 2 may be configured to communicate via the network 8 according to various communication channels, preferably a wireless communication channel such as via a cellular network (e.g. according to a standard protocol, such as 3G or 4G) or via a WiFi network.

The app installed on the mobile device and the application server 4 may be configured to assist a user with their smoking substitute device 10, based on information communicated between the smoking substitute device 10 and the app and/or information communicated between the app and the application server 4.

The charging station 6 (if present) may be configured to charge (and optionally communicate with) the smoking substitute device 10, via a charging port on the smoking substitute device 10. The charging port on the smoking substitute device 10 may be a USB port, for example, which may allow the smoking substitute device to be charged by any USB-compatible device capable of delivering power to the smoking substitute device 10 via a suitable USB cable (in this case the USB-compatible device would be acting as the charging station 6). Alternatively, the charging station could be a docking station specifically configured to dock with the smoking substitute device 10 and charge the smoking substitute device 10 via the charging port on the smoking substitute device 10.

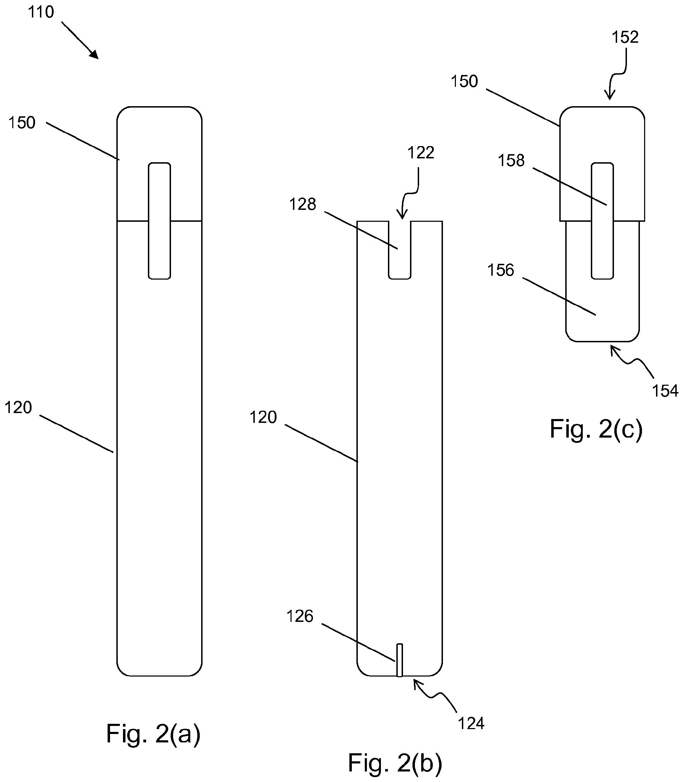

Fig. 2(a) shows an example smoking substitute device 110 for use as the smoking substitute device 10 in the system 1 of Fig. 1.

In this example, the smoking substitute device 110 includes a main body 120 and a consumable 150. The consumable 150 may alternatively be referred to as a "pod".

In this example, the smoking substitute device 110 is a closed system vaping device, wherein the consumable 150 includes a sealed tank 156 and is intended for one-use only.

Fig. 2(a) shows the smoking substitute device 110 with the main body 120 physically coupled to the consumable 150.

Fig. 2(b) shows the main body 120 of the smoking substitute device 110 without the consumable 150.

Fig. 2(c) shows the consumable 150 of the smoking substitute device 110 without the main body 120.

The main body 120 and the consumable 150 are configured to be physically coupled together, in this example by pushing the consumable 150 into an aperture in a top end 122 of the main body 120, e.g. with the consumable 150 being retained in the aperture via an interference fit. In other examples, the main body 120 and the consumable could be physically coupled together by screwing one onto the other, through a bayonet fitting, or through a snap engagement mechanism, for example. An optional light 126, e.g. an LED located behind a small translucent cover, is located a bottom end 124 of the main body 120. The light 126 may be configured to illuminate when the smoking substitute device 110 is activated.

The consumable 150 includes a mouthpiece (not shown) at a top end 152 of the consumable 150, as well as one or more air inlets (not shown in Fig. 2) so that air can be drawn into the smoking substitute device 110 when a user inhales through the mouthpiece. At a bottom end 154 of the consumable 150, there is located a tank 156 that contains e-liquid. The tank 156 may be a translucent body, for example.

The tank 156 preferably includes a window 158, so that the amount of e-liquid in the tank 156 can be visually assessed. The main body 120 includes a slot 128 so that the window 158 of the consumable 150 can be seen whilst the rest of the tank 156 is obscured from view when the consumable 150 is inserted into the aperture in the top end 122 of the main body 120.

In this present embodiment, the consumable 150 is a "single-use" consumable. That is, upon exhausting the e-liquid in the tank 156, the intention is that the user disposes of the whole consumable 150. In other embodiments, the e-liquid (i.e. aerosol former) may be the only part of the system that is truly "single-use". In such embodiments, the tank 156 may be refillable with e-liquid or the e-liquid may be stored in a non-consumable component of the system. For example, the e-liquid may be stored in a tank located in the device or stored in another component that is itself not single-use (e.g. a refillable tank).

The tank 156 may be referred to as a "clearomizer" if it includes a window 158, or a "cartomizer" if it does not.

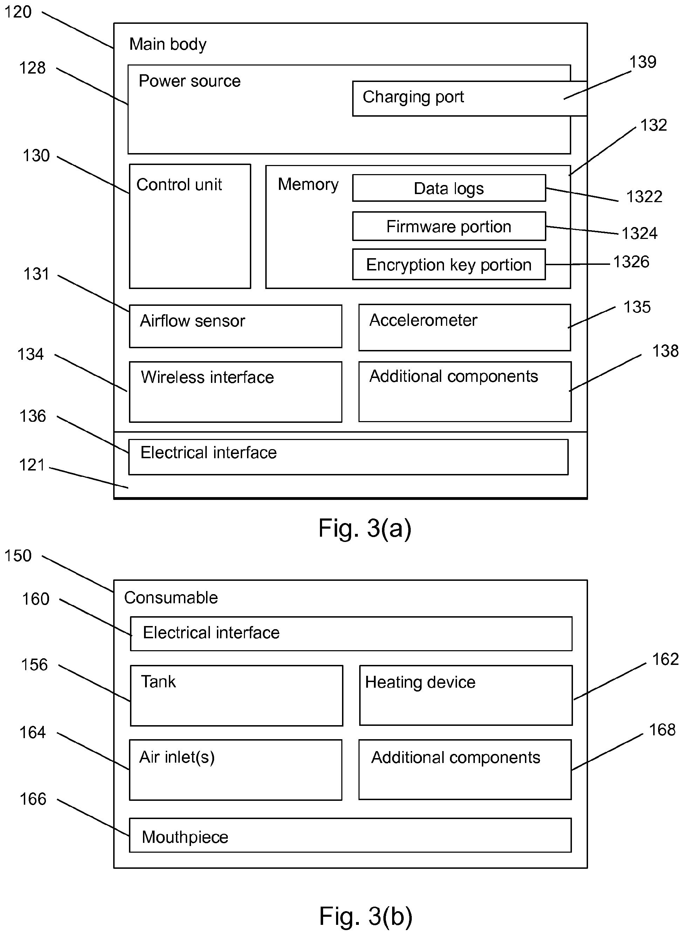

Fig. 3(a) is a schematic view of the main body 120 of the smoking substitute device 110.

Fig. 3(b) is a schematic view of the consumable 150 of the smoking substitute device 110.

As shown in Fig. 3(a), the main body 120 includes a power source 128, a control unit 130, an airflow sensor 131, a memory 132, a wireless interface 134, an electrical interface 136, and, optionally, an accelerometer 135 and/or one or more additional components 138.

The power source 128 is preferably a battery, more preferably a rechargeable battery. A charging port 139 may be provided for connecting the rechargeable battery to an external power source.

The control unit 130 may include a microprocessor, for example.

The memory 132 is preferably includes non-volatile memory, such as flash memory or the like.

The wireless interface 134 is preferably configured to communicate wirelessly with the mobile device 2, e.g. via Bluetooth. To this end, the wireless interface 134 could include a Bluetooth™ antenna. Other wireless communication interfaces, e.g. WiFi, are also possible.

The accelerometer 135 may function as a motion sensor to receive inputs for controlling the device.

The main body 120 comprises a coupling portion 121 that includes the electrical interface 136. The electrical interface 136 may include one or more electrical contacts. The electrical interface 136 may be located in, and preferably at the bottom of, the aperture in the top end 122 of the main body 120. When the main body 120 is physically coupled to the consumable 150, the electrical interface 136 may be configured to pass electrical power from the power source 128 to (e.g. a heating device of) the consumable 150 when the smoking substitute device 110 is activated, e.g. via the electrical interface 160 of the consumable 150 (discussed below). As an alternative to the charging port 139, the electrical interface may be configured to receive power from the charging station 6 when the main body 120 is not physically coupled to the consumable 150.

The electrical interface 136 of the main body 120 may include one or more electrical contacts. The electrical interface 136 may be located in, and preferably at the bottom of, the aperture in the top end 122 of the main body 120. When the main body 120 is physically coupled to the consumable 150, the electrical interface 136 may be configured to pass electrical power from the power source 128 to (e.g. a heating device of) the consumable 150 when the smoking substitute device 110 is activated, e.g. via the electrical interface 160 of the consumable 150 (discussed below). When the main body 120 is not physically coupled to the consumable 150, the electrical interface may be configured to receive power from the charging station 6.

The additional components 138 of the main body 120 may include the optional light 126 discussed above.

The additional components 138 of the main body 120 may, if the power source 128 is a rechargeable battery, include a charging port configured to receive power from the charging station 6. This may be located at the bottom end 124 of the main body 120. Alternatively, the electrical interface 136 discussed above is configured to act as a charging port configured to receive power from the charging station 6 such that a separate charging port is not required.

The additional components 138 of the main body 120 may, if the power source 128 is a rechargeable battery, include a battery charging control circuit, for controlling the charging of the rechargeable battery. However, a battery charging control circuit could equally be located in the charging station 6 (if present).

The airflow sensor 131 is configured to detecting airflow in the smoking substitute device 110, e.g. caused by a user inhaling through a mouthpiece 166 (discussed below) of the smoking substitute device 110. The smoking substitute device 110 may be configured to be activated when airflow is detected by the airflow sensor.

The additional components 138 of the main body 120 may include an actuator, e.g. a button. The smoking substitute device 110 may be configured to be activated when the actuator is actuated. This provides an alternative to the airflow sensor noted, as a mechanism for activating the smoking substitute device 110.

The additional components 138 of the main body 120 may include a reader configured to read information associated with the consumable from a machine readable data source included in (e.g. contained in the body of, or attached to) the consumable 150.

The reader may be configured to read information from the machine readable data source wirelessly, e.g. via electromagnetic waves or optically. Thus, for example, the machine readable data source included in the consumable 150 could be an RFID tag (in which case the reader included in the main body 120 may be an RFID reader) or a visual data source such as a barcode (in which case the reader included in the main body may be an optical reader, e.g. a barcode scanner). Various wireless technologies and protocols may be employed to allow the reader to wirelessly read information from a machine readable data source included in or attached to the consumable 150, e.g. NFC, Bluetooth, Wi-Fi, as would be appreciated by a skilled person.

For avoidance of any doubt, the reader (if present) may be configured to read information from the machine readable data source non-wirelessly, e.g. using a direct electrical connection between the main body 120 and consumable 150.

As shown in Fig. 3(b), the consumable 150 includes the tank 156, an electrical interface 160, a heating device 162, one or more air inlets 164, a mouthpiece 166, and, optionally, one or more additional components 168.

The electrical interface 160 of the consumable 150 may include one or more electrical contacts. The electrical interface 136 of the main body 120 and an electrical interface 160 of the consumable 150 are preferably configured to contact each other and therefore electrically couple the main body 120 to the consumable 150 when the main body 120 is physically coupled to the consumable 150. In this way, electrical energy (e.g. in the form of an electrical current) is able to be supplied from the power source 128 in the main body 120 to the heating device 162 in the consumable 150.

The heating device 162 is preferably configured to heat e-liquid contained in the tank 156, e.g. using electrical energy supplied from the power source 128. In one example, the heating device 162 may include a heating filament and a wick, wherein a first portion of the wick extends into the tank 156 in order to draw e-liquid out from the tank 156, and wherein the heating filament coils around a second portion of the wick located outside the tank 156. In this example, the heating filament is configured to heat up e-liquid drawn out of the tank 156 by the wick to produce an aerosol vapour.

The one or more air inlets 164 are preferably configured to allow air to be drawn into the smoking substitute device 110, when a user inhales through the mouthpiece 166.

The additional components 168 of the consumable 150 may include a machine readable data source, which may e.g. be contained in the body of, or attached to the consumable 150. The machine readable data source may store information associated with the consumable. The information associated with the consumable may include information concerning the content of the consumable (e.g. e-liquid type, batch number) and/or a unique identifier, for example.

The machine readable data source may be rewritable, e.g. a rewritable RFID chip, or read only, e.g. a visual data source such as a barcode. As indicated above, the additional components 138 of the main body 120 may include a reader configured to read information associated with the consumable from the machine readable data source.

In use, a user activates the smoking substitute device 110, e.g. through actuating an actuator included in the main body 120 or by inhaling through the mouthpiece 166 as described above. Upon activation, the control unit 130 may supply electrical energy from the power source 128 to the heating device 162 (via electrical interfaces 136, 166), which may cause the heating device 162 to heat e-liquid drawn from the tank 156 to produce a vapour which is inhaled by a user through the mouthpiece 166.

Of course, a skilled reader would readily appreciate that the smoking substitute device 110 shown in Figs. 2 and 3 shows just one example implementation of a smoking substitute device, and that other forms of smoking substitute device could be used as the smoking substitute device 10 of Fig. 1.

By way of example, a HNB smoking substitute device including a main body and a consumable could be used as the smoking substitute device 10 of Fig. 1, instead of the smoking substitute device 110. One such HNB smoking substitute device is the IQOS™ smoking substitute device discussed above.

As another example, an open system vaping device which includes a main body, a refillable tank, and a mouthpiece could be used as the smoking substitute device 10 of Fig. 1, instead of the smoking substitute device 110. One such open system vaping device is the blu PRO™ e-cigarette discussed above.

As another example, an entirely disposable (one use) smoking substitute device could be used as the smoking substitute device 10 of Fig. 1, instead of the smoking substitute device 110.

Embodiments of the present invention relate to the secure storage and/or transmission of different data types, for a smoking substitute device. The present inventors have recognised that it is desirable to control and manage the way in which particular types of data are stored, and separated from one another, and protected, within a smoking substitute device. In one example, the invention proposes encrypting three types of data using different encryption keys:
(1) firmware stored on the smoking substitute device,
(2) usage data recorded during operation of the smoking substitute device, and
(3) data to be transmitted wirelessly from the smoking substitute device to a mobile device or remote server.

In a preferred embodiment, each device has a unique set of three encryption keys for these data types. This is advantageous because the discovery of one key or a set of keys for a given smoking substitute device does not compromise the security of other smoking substitute devices.

Before the details of encryption techniques are discussed, the operation of the device is briefly discussed to provide an examples of usage data that may be recorded and stored on a smoking substitute device.

In use, the airflow sensor 131 is configured to transmit a detection signal to the control unit 130 when it detects an airflow in the airflow channel. The control unit 130 can then use that detection signal, either in isolation or in combination with other signals or other factors, to control operation of the smoking substitute device.

The signal emitted by the airflow sensor 131 may comprise a time component. For example, it may include an instantaneous time at which an airflow was initially detected, and/or a time period throughout which an airflow was detected, and/or a duration of an airflow - which would respectively correspond to an instantaneous time at which a user began an inhale action, the time period for which the inhale action lasted and the length of the inhale.

In the present embodiment, usage data may be obtained from any of the wireless interface 134, the accelerometer 135, or any of the optional additional components 138.

The wireless interface 134 may be configured to wirelessly communicate with a Bluetooth™ interface of another device (not shown), such as a mobile device, for example a smartphone. The Bluetooth™ interface can be configured to form a paired or bonded wireless communication link with the mobile device. It can further be configured to transmit data to the mobile device, and/or to an application running on the mobile device. The transmitted data may comprise usage data, e.g. telemetry data from components of the smoking substitute device, such as the wireless communication link itself. The transmitted data may also include activity/operation of the wireless communication link.

The usage data may comprise operational data for the smoking substitute device, for example voltage level readouts from the power source 128, which are indicative of remaining battery power or for example position and/or orientation data from the accelerometer, which are indicative of movements or physical actions made using the smoking substitute device. The mobile device may have an application running thereon that is configured to monitor received usage data from the smoking substitute device and to make calculations or determinations using the received data, and to transmit control signals or notifications to the smoking substitute device, based on the received data. For example, the application may be configured to send the smoking substitute device a notification when it is at low battery level, and possibly to send instructions regarding how various components within the smoking substitute device should be controlled at low battery level. As discussed below, however, for security reasons the application may not be able to decrypt encrypted data from the smoking substitute device on the mobile device. Instead, the encrypted data may be relayed by the mobile device to a remote server (which possesses a relevant decryption key), which decrypts the data and performs the necessary calculations. In this example, the raw (decrypted) usage data from the smoking substitute device may never exist in that form on the mobile device.

The accelerometer 135 may be configured to measure dynamic acceleration forces, in order to sense movement or vibrations. The outputs of the accelerometer 135 may be used to determine position factors and/or orientation factors such as tilt, tilt angle, and incline, as well as being used to determine actions or events such as rotation, vibration and collision. The accelerometer 135 may be a piezoelectric accelerometer or a capacitance accelerometer. The accelerometer 135 may comprise a three-axis model, to enable it to sense rotational tilt, as well as movement in a two-dimensional plane.

In this embodiment, the accelerometer 135 is configured to detect movement and collisions, and to provide one or more voltage outputs to the control unit 130, as a result of what it has detected. The accelerometer 135 can, for example, detect the action of the smoking substitute device being tapped against (i.e. relatively gently colliding with) a surface. When the user taps the smoking substitute device, the accelerometer 135 transmits a corresponding voltage signal to the control unit 130. The control unit 130 may store (at least temporarily) in memory a measure of the voltage signal, along with an indicator of the time at which it was received. This is an example of usage data that may be encrypted before being stored in the memory of the smoking substitute device in a manner discussed below.

If the smoking substitute device is currently paired with, or bonded to, a mobile device, it may also submit a signal to the mobile device, via the wireless communication link that has been established between them, regarding the detection that the accelerometer 135 has made. This can be very useful as the smoking substitute device may be preconfigured for a tap (or a plurality of taps) to form part of a sequence for the user to convey instructions to the smoking substitute device and/or to the connected mobile device or application. For example, there may be a predetermined sequence of hardware-related actions, which a user can make in order to reset a portion of the memory 132 of the smoking substitute device. Or the smoking substitute device may be used as a gaming input, for a game being running on a connected mobile device, wherein the accelerometer 135 is employed to control direction of movements being made by the user, within the game. The control unit 130 may record a log of the actions above. Such a log is an example of usage data that may be encrypted before being stored in the memory of the smoking substitute device in a manner discussed below.

The present inventors have observed that, in a smoking substitute device that is as sophisticated and functionally advanced as the smoking substitute device discussed above, the usage data may include sensitive data which it is desirable to protect from unauthorised access. Therefore, there is a need to apply security measures to the storage of, and to any transmission of, the data for a smoking substitute device.

We now discuss in more detail the use of three different encryption keys for three different respective data types.

A first data type (or facet, or datastream) that is protected using a first unique encryption key is the firmware. The firmware comprises the software instructions that can be implemented by the control unit 130 in order to control operation of components of the smoking substitute device, including those components discussed in detail hereabove. It is important to protect the firmware and to ensure that a non-authorised user cannot infiltrate or change the firmware, in order to ensure correct functioning of the smoking substitute device and to protect the user's data and ensure improved user peace of mind, particularly in the event that his or her smoking substitute device is lost or stolen.

For example, if a non-authorised user changed the firmware, he or she might be able to change the control instructions for controlling the heating device 162 of an inserted consumable 150, which could cause safety issues. For example, if an unauthorised user change the firmware, he or she might be able to change the address or other identifier of the mobile device, to which the wireless interface 134 of the smoking substitute device is linked for wireless communication, and as a result important operational data from the smoking substitute device may not reach the user's mobile device and corresponding important commands from the mobile device or application may not be transmitted to the user's smoking substitute device.

According to the present embodiment, the firmware is stored in a first portion 1324 of the memory 132. The first portion 1324 may also be referred to herein as the "firmware portion" of the memory 132. The firmware is stored in an encrypted form, which uses a first encryption key. The initial encryption and storage of the firmware may be carried out during manufacture and assembly of the smoking substitute device. The first encryption key is also stored in the memory 132 at this stage. The first encryption key is stored in a different portion of the memory to the encrypted firmware. In particular, the first encryption key is stored in a secure portion of the memory 132, which is referred to herein as an encryption key portion 1326. Herein the term "secure portion" may refer to computer storage that is configured to prevent unauthorised access. The secure portion may be a protected region of memory, e.g. under the control of a memory protection unit that forms part of the control unit 130. The memory protection unit may control access to the memory 132, and can prohibit access to the secure portion expect under certain circumstances. Alternatively, the secure portion may be provided on an entity that is a physically or logically separate from the first portion 1324. For example, the encryption key portion 1326 may be a suitably configured hardware security module, or the like.

The secure region protects the first encryption key against be read by an external device. The secure module may not be addressable by an external device, such as a mobile device with which the smoking substitute device can wirelessly communicate.

In operation, the control unit 130 is configured to decrypt the encrypted firmware with the first encryption key to enable the firmware to be executed. According to the present embodiment, the control unit 130 will thus has the ability to use the encryption key portion 1326 of the memory 132 to decrypt the encrypted firmware.

The second data type (or facet, or datastream) that is protected using a second unique encryption key is the usage data discussed above, which may take the form or a data log or a plurality of data logs 1322 stored in the memory 132. As will be understood from the detail descriptions above, various detectors and other functional components within the smoking substitute device provide operational data that can usefully be stored, monitored and in some cases used to make determinations regarding control and subsequent operations of the smoking substitute device. It is important to protect such data from being hacked, altered or deleted by a non-authorised user, in order to protect the integrity of the device's operation and to keep the user's information safe and private to him or her.

In the present embodiment, the memory 132 stores a plurality of data logs 1322 in a second portion thereof, which may be distinct from the firmware portion 1324 and the encryption key portion 1326. The data logs 1322 may include data pertaining to the operation of the power source 128, the air flow sensor 131, the accelerometer 135, the wireless interface 134 and the electrical interface 136 within the coupling portion 121 of the smoking substitute device. The data logs, or at least some of the data within those data logs, may be transmitted to a mobile device if the smoking substitute device is paired or bonded to a mobile device, as detailed further below.

The control unit 130 is configured to coordinate storage of data in the data logs, including determining what is to be stored, whether certain data should be combined to, for example, add a time component to a detection signal from a detector, and so on. The control unit is further configured to encrypt the data logs before storage in the memory 132. The control unit 130 may be configured to use a second encryption key stored within the encryption key portion 1326 for this purpose. Similarly to the firmware example above, the control unit 130 will not copy or store the second encryption key during this process, nor will it transmit the second encryption key to any other component within the smoking substitute device or to any device external to the smoking substitute device.

The second encryption key is different to the first encryption key. In the present embodiment the first and second encryption keys are stored along with a third encryption key detailed below in the encryption key portion 1326 of the memory 132, where in the encryption key portion 1326 is secure and non-readable by an external device. However, in some embodiments the first, second, and third encryption keys will be stored separately in first, second, and third respective secure regions of the memory 132.

In the present embodiment, all of the data within the data logs 1322 in the second portion of the memory 132 is encrypted using the second encryption key. However, in other embodiments the control unit may be configured only to apply the second encryption key to certain data logs 1322, for example if particular data logs 132 are deemed to be more sensitive and/or more useful than others. In other embodiments the control unit may be configured to apply multiple different encryption keys to multiple different respective data logs 132.

The third data type (or facet, or datastream) that is protected using a third unique encryption key is the information that is to be transmitted wirelessly from the wireless interface 134 of the smoking substitute device to a corresponding wireless interface of an external device, such as a mobile device, for example the mobile device 2 shown in Fig. 1 herein. In this embodiment, the wireless interface 134 is a Bluetooth™ interface. The smoking substitute device is configured to form a bonded wireless communication link, with a mobile device 2. The protocols for forming bonded Bluetooth™ links are generally well known, to the skilled reader, but are nonetheless described in brief below.

In this embodiment the user selects a mobile device 2 that he or she would like to be bonded to the smoking substitute device and usually downloads an application for managing the smoking substitute device, on to the mobile device 2, before the mobile device 2 is bonded to the smoking substitute device. However, it is not essential to download the application before the bonding process happens - it can be done afterwards, to control subsequent operation of the smoking substitute device, via the application running on the mobile device 2. The user activates the Bluetooth™ wireless interface 134 of the smoking substitute device, so that it emits an advertising message, seeking a mobile device 2 to bond with. The user will also activate the Bluetooth™ wireless interface of the mobile device 2, so that it can receive the advertising message from the smoking substituted device and respond thereto by identifying itself to the smoking substitute device. The two devices will then share pairing encryption keys with one another, wherein those pairing encryption keys are different to the first, second and third encryption keys detailed herein for encrypting data, and wherein the pairing encryption key that the smoking substitute device transmits to the mobile device 2 is stored in the memory 132 in a different location, away from the encryption key portion 1326 that stores the first second and third encryption keys.

When the smoking substitute device and the mobile device 2 have received and accepted one another's pairing encryption keys, a paired wireless communication link is formed between them. The paired link is an exclusive link, meaning that they will each direct subsequent wireless communication signals only to one another (unless the user instructs otherwise, either directly at the smoking substitute device or via the application - but those methods will not be discussed in detail herein.) The two devices will remember one another's pairing encryption keys and reuse them each time a wireless connection is to be formed between them - this remembering and re-using of the pairing encryption keys establishes a bonded wireless communication link between the two devices.

Once it has been bonded thereto, the smoking substitute device will begin to transmit wireless messages to, and receive wireless messages from, the mobile device 2. The wireless messages transmitted by the smoking substitute device can comprise commands or requests, issued to the mobile device 2 or to an application that is running on the mobile device 2. The wireless messages transmitted by the smoking substitute device can also comprise data relating to the usage of the smoking substitute device - i.e. comprise data that is stored in one or more of the data logs 1322. For example, in this embodiment the smoking substitute device is configured to regularly transmit a data log comprising data obtained from the airflow sensor 131, to the mobile device 2. That data obtained from the airflow sensor 131 can by accessed by the application, running on the mobile device 2, and used *inter alia* to monitor user smoking substitute behaviour patterns.

In this embodiment, before any information (including commands, requests and data log data) is transmitted by the wireless interface 134 of the smoking substitute device, to the mobile device 2, it is first encrypted using a third encryption key. The third encryption key is different to each of the first and second encryption keys. The control unit 130 may be configured to use a third encryption key stored within the encryption key portion 1326 for this purpose. Similarly to the firmware example above, the control unit 130 will not copy or store the third encryption key during this process, nor will it transmit the third encryption key to any other component within the smoking substitute device or to any device external to the smoking substitute device.

The third encryption key is not stored on, or known to, or directly accessible by, the mobile device 2. Therefore, the mobile device 2 will not (itself) be able to decrypt the information that it receives from the smoking substitute device. According to this embodiment, however, the mobile device 2 that the smoking substitute device is bonded with has an application installed thereon that is configured for managing the smoking substitute device. The application running on the mobile device 2 will be - as shown in Fig. 1 herein - configured to communicate with an application server 4 via a network 8, such as a Wi-Fi or 4G network. Moreover, in this embodiment, the server 4 is configured to store the first encryption key, second encryption key, and third encryption key. The server 4 stores the encryption keys securely, in a similar manner to how they are stored within the memory 132 of the smoking substitute device, so that the encryption keys themselves, as stored on the server 4, are not directly accessible by the application or the mobile device 2.

With the application installed on it, the mobile device 2 is configured to transmit information to and receive information from the server 4. Therefore the mobile device 2 can transmit the encrypted information received from the smoking substitute device to the server 4 and the server 4 can decrypt the encrypted information received from the mobile device 2, using the third encryption key stored on the server 4, and transmit the decrypted information to the mobile device 2. The mobile device 2 and/or the application running on the mobile device 2, can then store and/or use the information as appropriate.

According to a variant of this particular embodiment, the smoking substitute device itself may be able to communicate wirelessly with the application server 4 directly, not via the mobile device 2. In such an embodiment, the wireless interface 134 may be configured for network communication via a cellular or wireless data network (e.g. 3G, 4G, 5G, WiFi, etc.). In such a configuration, the smoking substitute device would encrypt information that is to be transmitted, using the third encryption key, and then transmit that encrypted information directly to the server 4, for it to be decrypted and transmitted to the application running on the mobile device 2.

In the present embodiment, the messages that the smoking substitute device receives from the mobile device 2, and/or directly from the server 4, should also be encrypted, using the third encryption key.

Such information may be generated at the server, whereby the mobile device 2 is used merely as a relay by which the application transmits information to the smoking substitute device. Alternatively, if the mobile device determines that a commend or other information is to be send to the smoking substitute device, the mobile device may notify the server 4, which may issue a corresponding information that is encrypted using the third encryption key. The control unit 130 may be configured to use a third encryption key stored within the encryption key portion 1326 for this purpose. The control unit can then process the information - be it a request, command, or other data - in the usual fashion.

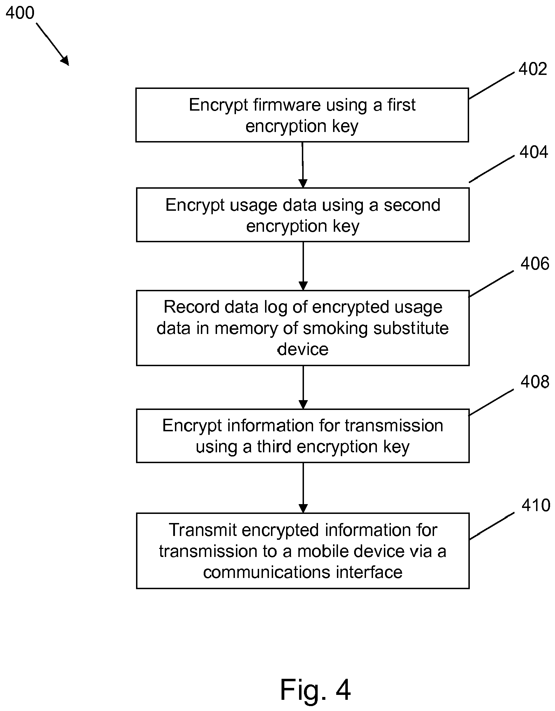

Fig. 4 is a flow chart depicting a method 400 of managing a smoking substitute device that is an embodiment of the invention. The method 400 relates to encryption operations performed by the control unit 130 of the smoking substitute device, in order to provide a secure data for storage and transmission architecture. It will be appreciated that the steps shown in Fig. 4 are not necessarily always done in the order shown - for example, data other than data log data, which is to be transmitted to a mobile device, may be encrypted using the third encryption key before other data is encrypted for storage in a data log. Moreover, it will be appreciated that encryption of data for storage in data logs and of information for wireless transmission (using the second and third encryption keys, respectively) will be carried out on a much more regular basis than encrypting of the firmware will be. However, there may be firmware updates, issued via the application and transmitted wirelessly via the mobile device 2 to the wireless interface 134 of the smoking substitute device, which the control unit 130 will have to decrypt with the third encryption key and apply using the first encryption key.

The encryption operations of the method can be summarised are as follows:
- At step 402, encrypt the firmware, that is stored in the firmware portion of the memory, using a first encryption key
- At step 404, encrypt the data relating to the use of the smoking substitute device using a second encryption key
- At step 406, record, in the memory a data log comprising the encrypted data relating to the usage of the smoking substitute device
- At step 408, encrypt information that is to be transmitted to a mobile device, via the communications interface, using a third encryption key
- At step 410, transmit the encrypted information to a mobile device, via the communications interface.

As a result of these encryptions - and of the corresponding decryptions, which are either performed by the server at the other end and/or by the smoking substitute device when it receives wireless messages - a secure means of storing and transmitting data is provided. It applies to different types of data including firmware, operational data and instructions and commands for and from other devices. Because the three encryption keys are different from one another and stored in a non-readable portion of the memory, and not stored anywhere else except at the server, a high level of data security is achieved. This is important to ensure the smoking substitute device can operate accurately and to avoid data breaches.

The embodiments described above are exemplary. Variations are possible. For example, according to an alternative embodiment, all the messages that the mobile device send to the smoking substitute device may not be encrypted. The application may be configured to determine whether certain messages are not sufficiently security sensitive as to require encryption. According to another variation, the smoking substitute device and mobile device may not have to be paired or bonded in order to communicate wirelessly, using messages encrypted as described herein.

Terms such as "first", "second" and "third" as used herein are intended to be illustrative and not to be limiting. The encryption keys should comprise (at least) three different encryption keys, each allocated to a different respective type of data, within a smoking substitute device. Moreover, each smoking substitute device should have its own unique set of three encryption keys.

Terms such as "firmware portion", "data logs" and "encryption key portion" as used herein are intended to be illustrative and not to be limiting. They are used to identify different sections, for storing different types of data, within the memory of the smoking substitute device. The actual logical and/or physical infrastructure and organisation of a memory of a smoking substitute device will depend *inter alia* on the particular make, model and type of the smoking substitute device.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A smoking substitute device comprising:
a control unit;
a communications interface; and
a memory configured to store firmware for controlling the smoking substitute device,
wherein the firmware is encrypted by a first encryption key,
wherein the control unit is configured to:
encrypt data relating to the use of the smoking substitute device using a second encryption key; and
record in the memory a data log comprising the encrypted data relating to the use of the smoking substitute device,
wherein the smoking substitute device is configured to transmit information to and receive information from a mobile device via the communications interface,
wherein the information transmitted to or received from the mobile device via the communications interface is encrypted using a third encryption key.

2. The smoking substitute device of claim 1, wherein the first encryption key, second encryption key and third encryption keys are different from one another

3. The smoking substitute device of claim 1 of claim 2, wherein the smoking substitute device is a first smoking substitute device within a plurality of smoking substitute devices according to claim 1 or claim 2 and wherein the first, second and third encryption keys for the first smoking substitute device are different to the first, second and third encryption keys for each of the respective other smoking substitute devices, within the plurality of smoking substitute devices.

4. The smoking substitute device of any preceding claim, wherein the first, second and third encryption keys are stored in a secure memory location within the smoking substitute device.

5. The smoking substitute device of any preceding claim, wherein the control unit is configured to run the firmware by decrypting the encrypted firmware stored in the memory using the first encryption key stored in the secure memory location.

6. The smoking substitute device of any preceding claim, wherein the information transmitted to the mobile device comprises the data relating to the use of the smoking substitute device encrypted by the second encryption key.

7. The smoking substitute device of any preceding claim, wherein the communications interface is further configured to transmit information directly to and receive information directly from a server, wherein the information transmitted to or received from the server via the communications interface is encrypted using the third encryption key

8. A system for managing a smoking substitute device, the system comprising:
a mobile device in network communication with a remote server; and
a smoking substitute device,
wherein the mobile device comprises:
a first control unit;
a first communications interface; and
a first memory,
wherein the smoking substitute device comprises:
a second control unit;
a second communications interface; and
a second memory configured to store firmware for controlling the smoking substitute device, wherein the firmware is encrypted by a first encryption key,
wherein the second control unit is configured to:
encrypt data relating to the use of the smoking substitute device using a second encryption key; and
record in the second memory a data log comprising the encrypted data relating to the use of the smoking substitute device,
wherein the mobile device and the smoking substitute device are in communication via the first communications interface and second communications interface to exchange information therebetween,
wherein the information exchanged between the mobile device and the smoking substitute device is encrypted using a third encryption key.

9. The system of claim 8, wherein the mobile device is configured to:
transmit encrypted information received from the smoking substitute device to the server; and
receive, from the server, encrypted data to be transmitted to the smoking substitute device.

10. The system of claim 9, wherein the information transmitted to the mobile device from the smoking substitute device comprises the data relating to the use of the smoking substitute device encrypted by the second encryption key, and wherein the server possesses:
the third encryption key to decrypt the transmitted encrypted information received by the mobile device from the smoking substitute device; and
the second encryption key to decrypt the data relating to the use of the smoking substitute device.

11. The system of any one of claims 8 to 10, wherein the second communications interface is further configured to transmit information directly to and receive information directly from the server, wherein the information transmitted to or received from the server via the communications interface is encrypted using the third encryption key.

12. The system of any one of claims 8 to 11, wherein the server stores the first encryption key, second encryption key, and third encryption key.

13. The system of claim 12, wherein the server is configured to transmit a firmware update to the smoking substitute device, and wherein the firmware update is encrypted with the first encryption key.

14. A method of managing a smoking substitute device, wherein the smoking substitute device comprises a control unit, a communications interface, and a memory configured to store firmware for controlling the smoking substitute device, the method comprising:
encrypting the firmware using a first encryption key;
encrypting data relating to use of the smoking substitute device using a second encryption key;
recording, in the memory, a data log comprising the encrypted data relating to the use of the smoking substitute device;
encrypting information that is to be transmitted to a mobile device, via the communications interface, using a third encryption key; and
transmitting encrypted information to a mobile device, via the communications interface.

15. The method of claim 14 further comprising:
transmitting, from the mobile device to a remote server, encrypted information received from the smoking substitute device; and
receiving, by the mobile device from the remote server, encrypted data to be transmitted to the smoking substitute device.
